# EUROPÄISCHE PATENTSCHRIFT

(11) **EP 0 770 324 B1**
(45) Veröffentlichungstag und Bekanntmachung des Hinweises auf die Patenterteilung: **14.05.2003**
(21) Anmeldenummer: 96890147.0
(22) Anmeldetag: 23.09.1996
(51) Int. Cl.: A01H 3/04

(54) **Verfahren zur Herstellung von Pflanzenkeimlingen**
Method for producing seedlings
Méthode pour produire des plantules

(30) Priorität: 09.10.1995 AT 166895
(43) Veröffentlichungstag der Anmeldung: 02.05.1997
(73) Patentinhaber: Vis-Vitalis Lizenz- und Handels AG, 5081 Anif (AT)
(72) Erfinder: Fuchs, Norbert, Mag., 5571 Mariapfarr (AT)
(74) Vertreter: Pawloy, Heinrich, Dr.

(56) Entgegenhaltungen:
- US-A- 2 051 460
- US-A- 4 130 964
- US-A- 4 240 817
- DATABASE COMPENDEX ENGINEERING INFORMATION, INC., NEW YORK, NY, US NERSON H ET AL: "EFFECTS OF SALINITY ON GERMINATION, SEEDLING GROWTH, AND YIELD OF MELONS" XP002081850 & IRRIG SCI DEC 1984, Bd. 5, Nr. 4, Dezember 1984, Seiten 265-273,
- DATABASE FSTA INTERNATIONAL FOOD INFORMATION SERVICE (IFIS), FRANFURT/MAIN, DE KHOKHAR S ET AL: "Antinutritional factors in moth bean (Vigna aconitifolia): varietal differences and effects of methods of domestic processing and cooking." XP002081851 & JOURNAL OF FOOD SCIENCE, Bd. 51, Nr. 3, 1986, Seiten 591-594, Dep. of Foods & Nutr., Haryana Agric. Univ., Hisar-125004, India
- DATABASE LIFESCI stn OKAI ET AL: "antitumor activity in an extract of Thai rice seedlings" XP002081852 & J. FERMENT. BIOENG., Bd. 76, Nr. 5, 1993, Seiten 367-370,
- Essentielle Spurenelemente in de Nahrung, W. Pfannhauser, S. 6

## Beschreibung

Die Erfindung betrifft ein Verfahren zu Herstellung von Pflanzenkeimlingen nach Ansprunch 1.

Das gegenwärtige Nahrungsmittelangebot sowie das Konsumverhalten ist gekennzeichnet durch Lebensmittel mit hohem Kaloriengehalt und gleichzeitig geringen Anteilen an Ballaststoffen und geringer Nährstoffdichte (geringen Gehalten an Vitaminen, Mengenelementen, Spurenelementen, bioaktiven Pflanzenstoffen, etc.). So wird mit den bevorzugt konsumierten Lebensmitteln, wie Weißmehlprodukten (Brot, Gebäck, Eierteigwaren, Feingebäck), Zucker und zuckerhaltigen Lebensmitteln (Süßigkeiten, zuckerhaltigen Limonaden), Fastfood-Gerichten und Lebensmitteln mit hohem Anteil an tierischen Proteinen der Bedarf an Ballaststoffen, vor allem aber an Vitaminen, Mengen- und Spurenelementen, nur unzureichend gedeckt. Die Folge dieses Konsumverhaltens ist ein ständig steigender Anteil ernährungsbedingter und ernährungsabhängiger Erkrankungen, wie Übergewicht, chronische Stuhlbeschwerden, Bluthochdruck mit erhöhten Blutfett- und Triglyceridwerten, Zuckerstoffwechselstörungen, Leber- und Gallenerkrankungen, Durchblutungsstörungen, Erkrankungen der Verdauungsorgane, Karies, rheumatische Erkrankungen, Gicht, Hauterkrankungen, Allergien und Störungen der Immunabwehr.

Parallel zur Entvitaminisierung und Entmineralisierung häufig konsumierter Grundnahrungsmittel (z.B. durch Raffination verschiedener Getreidemehle und Pflanzenöle) ist statistisch eine stetig zunehmende Kontamination von Grundnahrungsmitteln mit Fremd- und Schadstoffen zu verzeichnen (Organohalogenverbindungen, Agrochemikalien, wie Pestizide, Wachstumsregulatoren, Keimhemmern und Düngemitteln, Schwermetalle, Arzneimittelrückstände, Pflanzengifte, etc.).

Nicht zuletzt werden Lebensmittelgrundstoffe aus produktionstechnischen Gründen mit Farbstoffen, Konservierungsstoffen, Antioxidationsmittel, Emulgatoren, Stabilisatoren, Dickungsmitteln, Geliermitteln, modifizierten Stärken, Säuerungsmitteln, Säureregulatoren, Trennmitteln, Überzugsmitteln, Tauchmassen, Geschmacksverstärkern, Aromastoffen, Zuckeraustauschstoffen, künstlichen Süßstoffen und sonstigen technologischen Stoffen versetzt.

Um dieser Verschlechterung der Ernährungssituation bzw. diesen negativen Entwicklungen des Ernährungsverhaltens entgegenzuwirken, sind immer mehr Konsumenten daran interessiert, diese Ernährungsgewohnheiten zu verändern und vermehrt naturbelassenere Lebensmittel zu konsumieren, die eine ausreichende Versorgung mit Ballaststoffen, Mineral- und Spurenelementen, Vitaminen, pflanzlichen Proteinen, etc. ermöglichen (sogenannte "Vollwerternährung").

Die Vollwerternährung besteht vorwiegend aus pflanzlichen Lebensmitteln aus ökologischer Produktion, wobei isolierte oder raffinierte Produkte möglichst vermieden werden.

Keimlinge erfüllen die Prinzipien der Vollwerternährung, sowohl nach ernährungsphysiologischen als auch nach ökologischen Gesichtspunkten. Im Vergleich zum ungekeimten Samen hat der Keimling eine bessere Proteinqualität, einen höheren Gehalt an mehrfach ungesättigten Fettsäuren, eine verbesserte Bioverfügbarkeit essentieller Mineralstoffe und einen höheren Vitaminund Ballaststoffgehalt. Weiters nimmt eine Vielzahl negativ zu bewertender Inhaltsstoffe im Samen, wie Trypsininhibitoren, Hämagglutinine, Saponine, Blähsubstanzen, etc. mit zunehmender Keimdauer ab.

Keimlinge stellen daher eine wertvolle Bereicherung der Nahrung dar, insbesondere, da sie beispielsweise im Vergleich zu Gemüsesorten preiswert, stets frisch, saisonunabhängig, ballaststoffreich, vitamin- und mineralstoffreich und zudem schmackhaft und gut bekömmlich sind.

Im allgemeinen bezeichnet man die aus einem Samenkorn wachsende Pflanze innerhalb der ersten Keimtage als Keimling. Der vorgebildete Keimling im Samen befindet sich vor der Keimung in einem Ruhezustand, in dem alle Stoffwechselvorgänge auf ein Minimum reduziert sind und kein Wachstum erfolgt. Der Quellprozeß beginnt mit der Aufnahme von Wasser in den Samen und bewirkt dadurch die Aktivität der Stoffwechselvorgänge. Die bis dahin sauerstoffundurchlässige Samenschale wird atmungsaktiv, Phytohormone (insbesondere Giberellinsäure) werden im Embryo synthetisiert, die wiederum die Sythese spezifisch wirksamer Enzyme stimulieren. Diese Enzyme bauen die im Samen in artspezifischen Mengen gespeicherten Reservestoffe ab (Meier-Ploeger "Die Bedeutung von Sprossen und Keimen in der Vollwerternährung", Ernährung/Nutrition (6) (1990), 317-323).

Über die Veränderungen des Gehaltes an Inhaltsstoffen des Keimlings im Vergleich zum Samen sind zahlreiche Untersuchungen durchgeführt worden, wobei diese Untersuchungen häufig widersprüchlich sind. Inwieweit diese Diskrepanzen auf Unterschiede in der Beschaffenheit des Ausgangsmaterials, der Keimbedingungen oder der Methodik der Nährwertbestimmung beruhen, ist unklar (Harmuth-Hoene et al., "Der Einfluß der Keimung auf den Nährwert von Weizen, Mungobohnen und Kichererbsen", Z. Lebensm. Unters. Forsch., 185 (1987), 386-393).

Auch die Angaben über die Veränderungen des Mineralstoffgehaltes in Keimlingen sind widersprüchlich. Einigkeit scheint darüber zu herrschen, daß in Abhängigkeit der Löslichkeit der Mineralstoffe sehr verschiedene Verluste an Mineralstoffen während der Keimung auftreten können. Es wurde daher von verschiedenen Autoren eine Abnahme von Eisen in der Höhe von 9 bis 21 %, von Kalium von 27 % und von Kupfer zwischen 12 und 17 % beobachtet (Hartmuth-Hoene (1987)).

Weiters wurden auch hohe Verluste an Kalzium und Magnesium im Verlaufe der Keimung berichtet.

Die vorliegende Erfindung stellt sich daher die Aufgabe, Keimlinge zur Verfügung zu stellen, welche ernährungsphysiologisch gegenüber herkömmlichen Keimlingen verbessert sind.

Diese Aufgabe wird erfindungsgemäß durch Keimlinge mit einem gegenüber der Keimung in Leitungswasser erhöhten Elektrolytgehalt gelöst, die durch Keimung in einer Elektrolytlösung, enthaltend 1 mg/l oder mehr Zink-, Eisen-, Kalium- und/oder Magnesium-Ionen, 0,5 mg/l oder mehr Kupfer-, Mangan-, Strontium- und/oder Lithium-Ionen und 0,1 mg/l oder mehr Selen-, Molybdän-, Chrom-, Arsen-, Vanadium- und/oder Kobalt-Ionen, erhältlich sind.

Die erfindungsgemäßen Keimlinge weisen einen im Vergleich zu herkömmlich aufgekeimten Samen um mindestens 10 bis 20 %, vorzugsweise um mindestens das 1,5- bis 3-fache, insbesondere um mindestens das 5- bis 10-fache, erhöhten Gehalt an einem oder mehreren Elektrolyten, vorzugsweise an Zink, Eisen, Kalium, Magnesium, Kupfer, Mangan, Strontium, Selen, Molybdän, Chrom, Arsen, Vanadium und/oder Kobaltionen, auf.

Bislang traten bei der herkömmlichen Samenkeimung, bei welcher die Samen in destilliertem Wasser oder in Leitungswasser zur Keimung gebracht worden sind, immer zum Teil erhebliche Verluste an diesen für die Ernährung wichtigen Bestandteilen auf. Diese Verluste waren, wie sich bei den Untersuchungen zu der vorliegenden Erfindung herausstellte, sowohl durch den beginnenden Stoffwechselprozeß des Pflanzenkeimlings selbst bedingt, aber auch durch die Natur des Quellmittels Wasser, welches zu einer zusätzlichen Elektrolytauslaugung des Keimlings beitrug, da, im Gegensatz zum Ruhezustand (Samen), die Schale des Keimlings einer Elektrolytauslaugung sehr wohl zugänglich ist.

Es hat sich weiters gezeigt, daß die erfindungsgemäßen elektrolytangereicherten Keimlinge nicht nur eine höhere Konzentration an Mineralstoffen aufweisen, sondern, bedingt durch den erhöhten Mineralstoffgehalt, auch ganz allgemein im Hinblick auf ihre Inhaltsstoffe verbessert sind, beispielsweise einen erhöhten Vitamingehalt aufweisen.

Die erfindungsgemäßen Keimlinge werden hergestellt, indem die keimfähigen Samen in eine Elektrolytlösung, enthaltend 1 mg/l oder mehr Zink-, Eisen-, Kalium- und/oder Magnesium-Ionen, 0,5 mg/l oder mehr Kupfer-, Mangan-, Strontium- und/oder Lithium-Ionen und 0,1 mg/l oder mehr Selen-, Molybdän-, Chrom-, Arsen-, Vanadium- und/oder Kobalt-Ionen, eingebracht werden und die Keimlinge in der Elektrolytlösung bei einer geeigneten Temperatur während einer Zeitdauer, die ausreicht, um in den Keimlingen eine Elektrolytanreicherung zu erzielen, inkubiert werden.

Es war überraschend, daß unter Verwendung der erfindungsgemäßen Elektrolytlösung, also einer Lösung, die im Gegensatz zu den herkömmlichen Keimungslösungen (Leitungswasser bzw. destilliertes oder sterilisiertes Wasser) erhöhte Ionenkonzentration beinhaltet, die im Zuge der Keimung auftretenden Elektrolytverluste ausgeglichen werden konnten bzw. sogar durch einen Elektrolytfluß aus der Keimungslösung in die Keimlinge ins Gegenteil verkehrt werden konnte und somit Keimlinge entstanden, die z.T. sogar einen gegenüber dem Samen erhöhten Gehalt an Elektrolyten aufweisen.

Unter Elektrolytlösung wird im weiteren eine wässerige Lösung verstanden, welche mit einem oder mehreren Elektrolyten wie nachstehend definiert versetzt bzw. angereichert ist.

Die Ionenkonzentration der Elektrolytlösung soll beim erfindungsgemäßen Herstellungsverfahren um mindestens 10 bis 20 % über derjenigen von herkömmlichem Leitungswasser liegen, vorzugsweise ist die Ionenkonzentration der Elektrolytlösung zumindest in Bezug auf Eisen- und/oder Kupfer- und/oder Mangan- und/oder Strontiumund/oder Lithium- und/oder Molybdän-Ionen zweimal so hoch wie die von herkömmlichem Leitungswasser, besonders bevorzugt mindestens fünfmal so hoch, insbesondere mindestens zehnmal so hoch.

Die geeignete Temperatur zur Durchführung der Keimung ist selbstverständlich von Samenart zu Samenart verschieden. Prinzipiell ist die Keimungstemperatur, welche im Stand der Technik für die jeweilige Samenart beschrieben ist, auch für das erfindungsgemäße Verfahren anzuwenden. Vorzugsweise liegt diese Temperatur zwischen 10 und 50°C, insbesondere zwischen 20 und 30°C.

Die Zeitdauer, um in den Keimlingen eine ausreichende Elektrolytanreicherung zu erzielen, ist ebenfalls von Keimlingsart zu Keimlingsart unterschiedlich, es hängt auch davon ab, welche Elektrolytwerte im Keimling erreicht werden sollen. Auch hier dienen für eine bestimmte Art. die Keimungsdauern, welche im Stand der Technik beschrieben sind, als Richtwerte, bevorzugterweise wird daher die Keimung während einer Zeitdauer von etwa 12 bis 120 Stunden, insbesondere etwa 60 bis 100 Stunden, durchgeführt.

Es versteht sich, daß sowohl die Keimungstemperatur als auch die Keimungsdauer von einem Fachmann ohne weiteres durch einfache Versuche für jedes System optimiert werden und für bestimmte Arten durchaus auch über oder unter den oben angegebenen Richtwerten liegen kann.

Als bevorzugte Keimlinge sind erfindungsgemäß Keimlinge von gängigen pflanzlichen Nahrungsmitteln vorgesehen, insbesondere Keimlinge von Hülsenfrüchten und Getreidesamen. Besonders bevorzugte Keimlinge sind daher Weizen-, Buchweizen-, Quinoa-, Mungobohnen-, Bockshornklee-, Rettich-, Alfalfa-, Mais-, Kürbis-, Roggen-, Gerste-, Reis-, Adzuki-Bohnen-, Erbsen-, Hirse-, Kichererbsen-, Kresse-, Leinsamen-, Linsen-, Senf-, Sesam-, Sojabohnen-, Sonnenblumen- und Amaranthkeimlinge.

Die beim erfindungsgemäßen Verfahren verwendete Elektrolytlösung enthält gemäß einer bevorzugten Ausführungsform 10 mg/l oder mehr, insbesondere 50 mg/l oder mehr Zink- und/oder Eisen- und/oder Kalium- und/oder Magnesium-Ionen, 5 m/l oder mehr, insbesondere 25 mg/l oder mehr Kupfer- und/oder Mangan- und/oder Strontrium- und/oder Lithium-Ionen, 1 mg/l oder mehr, insbesondere 5 mg/l oder mehr Selen- und/oder Molybdän- und/oder Chromund/oder Arsen- und/oder Vanadium- und/oder Kobalt-Ionen mit der Maßgabe, daß sich die Ionenkonzentration der Elektrolytlösung in zumindest einer Ionenspezies von der Ionenkonzentration in Leitungswasser um mindestens 10 bis 20 % unterscheidet.

Eine besonders bevorzugte Elektrolytlösung enthält zumindest 0,5 mg/l Kupfer- und/oder 1 mg/l Zink- und/oder 0,1 mg/l Kobalt- und vorzugsweise mindestens 0,1 mg/l Molybdän- und/oder 0,5 mg/l Lithium- und/oder 1 mg/l Selen- und/oder 1 mg/l Vanadium-Ionen.

Die erfindungsgemäßen Elektrolyt-angereicherten Keimlinge können nach ihrer Herstellung je nach Verwendungszweck gewaschen, getrocknet und gegebenenfalls für den Verkauf geeignet weiterverarbeitet werden. Besonders bevorzugt ist die Aufbereitung der erfindungsgemäßen Keimlinge zu Frischkost, zu Brotaufstrichen, zu Backwaren oder zu Snack-artigen Lebensmitteln oder Nahrungsergänzungen in Form von Mueslis, Kautabletten, Kapseln oder Liquida.

Die Erfindung wird in den nachstehenden Beispielen und den dazugehörigen Zeichnungen, auf die sie jedoch nicht beschränkt sein soll, noch weiter erläutert.

Es zeigen:
Fig. 1 die Anreicherung von Spurenelementen während der Keimung von Weizen;
Fig. 2 die Anreicherung von Spurenelementen während der Keimung von Buchweizen,
Fig. 3 die Anreicherung von Spurenelementen während der Keimung von Quinoa; und
Fig. 4 die chromatographische Vitamin B1-Bestimmung in einer Standardlösung mit 104 µg/g Thiaminhydrochlorid und in der Probe BoS6 (Bockshornkleekeimlinge nach 3 Tagen Keimzeit).

### Beispiele:

### 1. Anreicherung von Spurenelementen während der Keimung von Weizen, Buchweizen und Quinoa.

### 1.1. Keimung

Für die Keimversuche wurden keimfähige Samen von Weizen (Triticum aestivum), Buchweizen (Fogpyrum esculentum) und Quinoa (Chenopodium quinoa) verwendet. Ca. 90 g der drei verschiedenen Getreidesamen wurden jeweils mit fünf unterschiedlichen Lösungen zur Keimung gebracht: (1) zweifach destilliertes Wasser, (2) Leitungswasser, (3) Elektrolytlösung 1, (4) Elektrolytlösung 2 und (5) Elektrolytlösung 3 (siehe Tabelle 1). Zur Herstellung der Elektrolytlösungen wurden nur p.a. Chemikalien und zweifach destilliertes Wasser verwendet.

**Tabelle 1:**

| Spurenelementkonzentrationen der verwendeten Elektrolytlösungen | | | | | | | |
|---|---|---|---|---|---|---|---|
| | Elektrolytlösungen | | | Element | Elektrolytlösungen | | |
| Substanz | c [mg/l] | | | | c [mg/l] | | |
| | 1 | 2 | 3 | | 1 | 2 | 3 |
| Zinksulfat x7H₂O | 4,40 | 44,0 | 220 | Zn | 1,0 | 10 | 50 |
| Ammonium-Eisen-III-citrat | 8,95 | 89,5 | 447 | Fe | 1,0 | 10 | 50 |
| Manganchlorid | 1,48 | 14,8 | 74 | Mn | 0,5 | 5 | 25 |
| Kupfergluconat | 3,57 | 35,7 | 178 | Cu | 0,5 | 5 | 25 |
| Natriumselenat | 0,24 | 2,4 | 12 | Se | 0,1 | 1 | 5 |
| Natriummolybdat | 0,25 | 2,5 | 12,5 | Mo | 0,1 | 1 | 5 |
| Chrom-III-chlorid | 0,51 | 5,1 | 25,5 | Cr | 0,1 | 1 | 5 |
| Strontiumloctet | 1,69 | 16,9 | 84,5 | Sr | 0,5 | 5 | 25 |
| Lithiumcarbonat | 2,68 | 26,8 | 134 | Li | 0,5 | 5 | 25 |
| Dinatriumarsenat | 0,56 | 5,6 | 28 | As | 0,1 | 1 | 5 |
| Ammonvanadat | 0,23 | 2,3 | 11,5 | V | 0,1 | 1 | 5 |
| Kobaltchlorid x6H₂O | 0,40 | 4,0 | 20 | Co | 0,1 | 1 | 5 |

Vor der eigentlichen Keimphase wurde der Weizensamen 12 Stunden und der Quinoasamen 8 Stunden in den entsprechenden Lösungen eingeweicht. Der Buchweizensamen wurde ohne vorheriges Einweichen verwendet.

Die Keimung erfolgte bei Raumtemperatur (19 bis 21°C) und normalen Tag-Nacht-Lichtverhältnissen in handelsüblichen Keimgeräten, bestehend aus durchsichtigen, übereinander angeordneten Plastikschalen mit Ablaufvorrichtung. Die Gesamtkeimdauer (Einweichzeit + Keimzeit) betrug für Weizen und Quinoa 96 Stunden, für Buchweizen 72 Stunden. Während der Keimung wurden die Keimlinge zweimal täglich mit den entsprechenden Lösungen (250 ml/90 g) gespült. Nach der Ernte wurden alle Keimlinge gründlich mit zweifach destilliertem Wasser gespült (3 x mit ca. 800 ml) und aliquotiert. Ein Teil der Probe wurde sofort in Plastiksäckchen abgefüllt und bei -18°C tiefgefroren. Der andere Teil der Probe wurde vor dem Einfrieren noch einmal mit 70°C heißem Leitungswasser nachgespült (3 x mit ca. 800 ml) (siehe Tabelle 2).

**Tabelle 2:**

| Probenverzeichnis | | | |
|---|---|---|---|
| | Weizen | Buchweizen | Quinoa |
| Samen | WS0 | BS0 | QS0 |
| Keimung mit destilliortem Wasser | WS1 | BS1 | QS1 |
| Keimlinge heiß gewaschen | WS1H | BS1H | QS1H |
| Keimung mit Leitungswasser | WS2 | BS2 | QS2 |
| Keimlinge heiß gewnschen | WS2H | BS2H | QS2H |
| Keimung mit Elektrolytlösung 1 | WS3 | BS3 | QS3 |
| Keimlinge heiß gewaschen | WS3H | BS3H | QS3H |
| Keimung mit Elektrolytlösung 2 | WS4 | BS4 | QS4 |
| Keimlinge heiß gewaschen | WS4H | BS4H | QS4H |
| Keimung mit Elektrolytlösung 3 | WS5 | BS5 | QS5 |
| Keimlinge heiß gewaschen | WS5H | BS5H | QS5H |

### 1.2. Probenvorbereitung

Die Proben wurden in einer Gefriertrocknungsanlage (CHRIST ALFA 1-4 mit Anlagesteuerung LDC-1M) wie folgt getrocknet: Die Keimlinge (jeweils ca. 50 g) wurden zuerst bei -30°C (Tiefkühltruhe) und anschließend bei -45°C (Kondensatorraum der Gefriertrocknungsanlage) tiefgefroren. Danach erfolgte die Haupttrocknung bei -15°C und einem Druck von 0,31 mbar (Sicherheitsdruck 5 mbar). Nach 36 h wurde die Trocknungstemperatur (Temperatur des Probentellers der Anlage) auf 0°C erhöht. Nach einer Gesamtzeit von 72 h waren die Proben völlig trocken und für die weitere Probenvorbereitung verwendbar. Während des gesamten Trocknungsprozesses wurde sichergestellt, daß die Proben nie aufgetaut worden sind. Die trockenen Keimlinge wurden anschließend in einer kontaminationsfreien Analysenmühle (Retsch ZM 1000, mit Titanrotor und Titansieb; Korngröße 0,25 mm) homogenisiert.

### 1.3. Bestimmung der Spurenelementkonzentrationen mit ICP-MS und GFAAS

### Mineralisierung

Ungefähr 200 mg Probe wurden in ein Teflongefäß exakt eingewogen, mit 3 ml HNO₃ bidest. und 0,5 ml H₂O₂ versetzt und im Mikrowellenaufschlußgerät (MLS, 1200 mega, ausgestattet mit einem Rotor für 10 Proben) mit folgendem Energieprogramm mineralisiert: 2 min 250W, 0,5 min 0W, 10 min 250W, 0,5 min 0W, 5 min 450W, 0,5 min 0W, 7 min 600W, 1 min 500W. Nach dem Abkühlen wurden die Aufschlußlösungen in Maßkolben (10 ml) übergeführt und mit H₂O nanopur aufgefüllt. Von jeder Probe wurden zwei Aufschlüsse gemacht. Jede Aufschlußlösung wurde dreimal vermessen, wobei die gemessenen Konzentrationen mit den Konzentrationen eines Aufschluß-Blanks korrigiert wurden. Parallel zu den Proben wurden zur Überprüfung der Richtigkeit der Analyse zwei Standardreferenzmaterialien mit ähnlicher Matrixzusammensetzung (SRM-NIST 1575 Pine Needles und SRM-BCR 62 Olive Leaves) analysiert.

### Messung mit GFAAS

Die Selen- und Arsenkonzentrationen wurden mit einem Hitachi Z9000 GFAAS bestimmt. Die experimentellen Bedingungen sind in Tabelle 3 zusammengefaßt. Quantifiziert wurde mittels externer Eichkurven.

**Tabelle 3:**

| Experimentelle Parameter zur Bestimmung der Se-und As-Konzentrationen mit GFAAS | | | | | | | |
|---|---|---|---|---|---|---|---|
| | Se | | As | | | Se/As | |
| Lampenstrom | 12mA | | 12 mA | | Standard 1 | | 0 µg/l |
| Wellenlänge | 195 nm | | 196 | | Standard 2 | | 20 µg/l |
| Spalt | 1,2 nm | | 1,3 nm | | Standard 3 | | 50 µg/l |
| Kuvette | Tube | | Tube | | Standard4 | | 100 µg/l |
| Modifizierer | 2%Ni(NO₃)₂ | | 2%Ni(NO₃)₂ | | Standard 5 | | 200 µg/l |
| Volumen | 20 µl | | 20 µl | | | | |

| Temperatur-Programm | | | | | | | |
|---|---|---|---|---|---|---|---|
| | | Se | | | | As | |
| Trocken | 80°C | 120°C | 30 s | 80°C | | 120°C | 30s |
| Trocken | 120°C | 400°C | 10 s | 120°C | | 500°C | 20 s |
| Asche | 700°C | 700°C | 30 s | 800°C | | 800°C | 30 s |
| Atom | 2400°C | 2400°C | 10 s | 2000°C | | 2000°C | 10 s |
| Rein | 3000°C | 3000°C | 5 s | 3000°C | | 3000°C | 5 s |
| Trägergas | | 200 ml/min | | | | 200 ml/min | |
| unterbr. Gas | | 30 ml/min | | | | 30 ml/min | |

### Messung mit ICP-MS

Die Konzentrationen der Spurenelemente Cr, Cu, Ni, Pb, Sr, Li, Fe, Zn, Mn, Cd, Co, Mo und V wurden mit einem ICP-MS der Firma Fisons, Typ PlasmaQuad II+ durchgeführt. Die experimentellen Parameter sind in Tabelle 4 zusammengefaßt. Vor der Messung wurden die Aufschlußlösungen 1 : 5 mit H₂O verdünnt. Um interne Geräteschwankungen zu korrigieren, wurde den Aufschlußlösungen und den Standardlösungen 50 ppb Indium, 50 ppb Gallium und 50 ppb Rhenium als interner Standard zugesetzt. Quantifiziert wurde mittels externer Eichkurven.

**Tabelle 4:**

| Experimentelle Parameter zur Bestimmung der Spurenelementkonzentrationen mit ICP-MS | | | |
|---|---|---|---|
| | ICP-MS PlasmaQuad Π+ | | |
| rf Leistung | 1,3 kW | Zeit/Sweep | 1,22 s |
| Colling-Gas | 13,5 l/min | Verweilzeit | pulse count mode 320µs |
| Hilfsgas | 1,1 l/min | Datenaufnahme | peak jump mode |
| Nebulizer-Gas | 0,88 l/min | Aufnahmezeit | 60 s |
| Nebulizer | Meinhard Tr-30-A3 | Meßzeit | 3 x 60 s |
| Spray -Kammer | double pass Scott-typ (-2°C) | Waschzeit | 60 s |
| Sampling-Cone | Nickel, orifice 1,00 mm | | |
| Skimmer-Cone | Nickel, orifice 0,75 mm | Standard 1 | blank |
| Vakuum:Expansion | 1,6 mbar | Standard 2 | 5 µg/l |
| Vakuum:Intermediate | 1,0 x 10⁻⁴ mbar | Standard 3 | 10 µg/l |
| Vakuum: Analyzer | 2,1 x 10⁻⁶ mbar | Standard 4 | 50 µg/l |

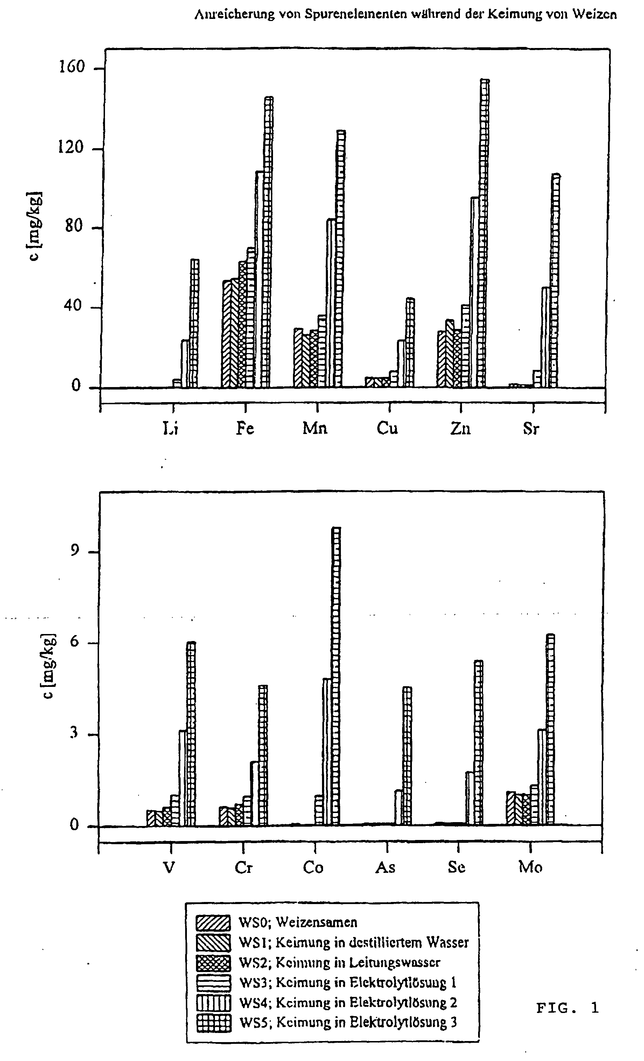

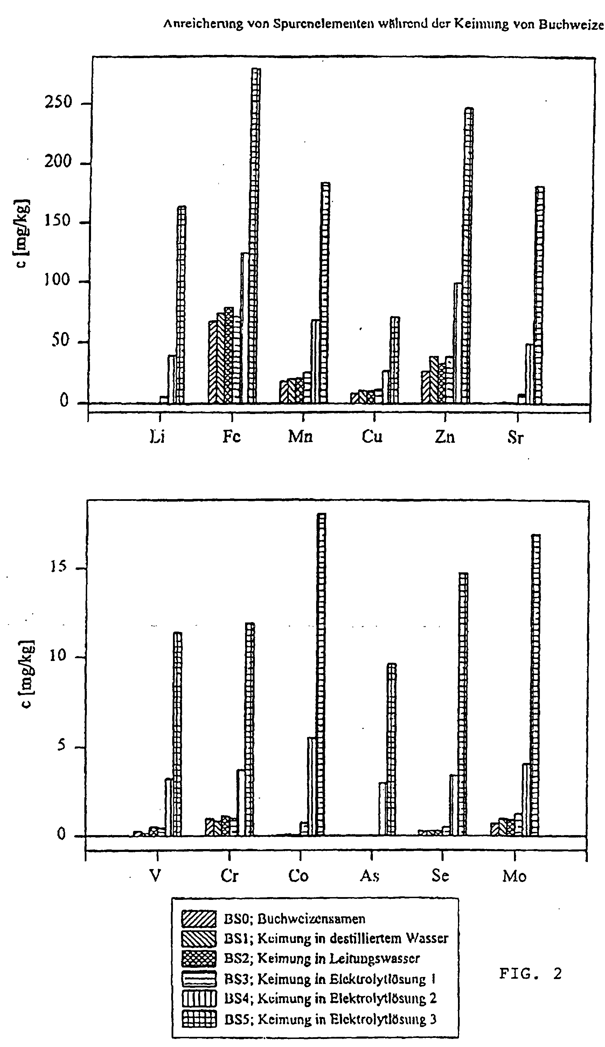

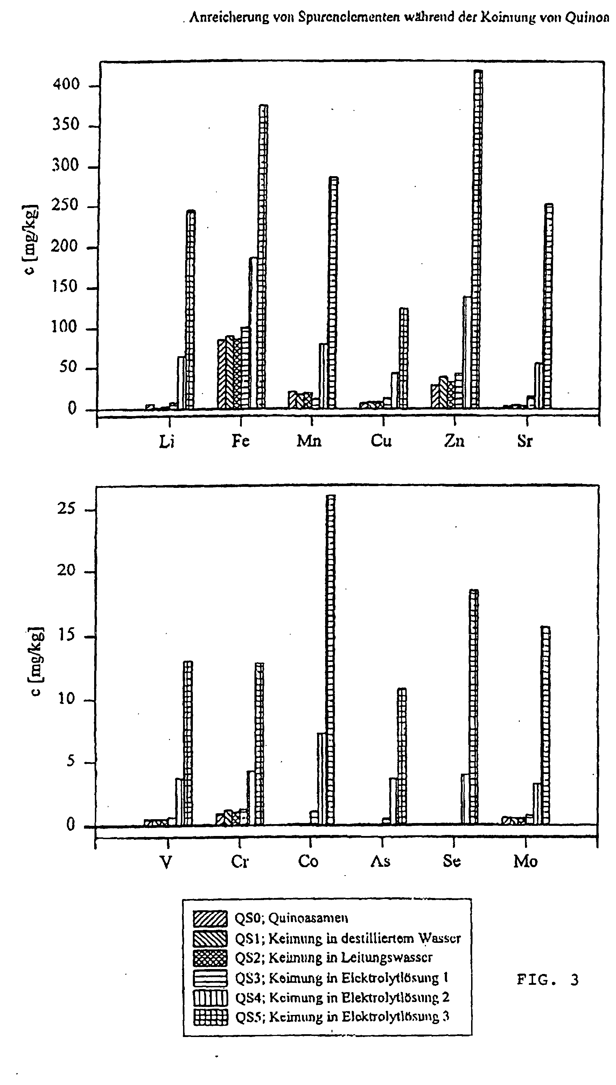

Die Ergebnisse sind in Tabellen 5 und 6 samt Fig. 1-3 zusammengefaßt. Daraus ist deutlich ersichtlich, daß die Keimung der Samen in einer Elektrolytlösung zu einer deutlichen Erhöhung des Elektrolytgehaltes der Keimlinge führen, während die Keimung in destilliertem Wasser bzw. Leitungswasser bei vielen Ionenspezies eine Erniedrigung der Konzentration dieser Ionenspezies mit sich brachte.

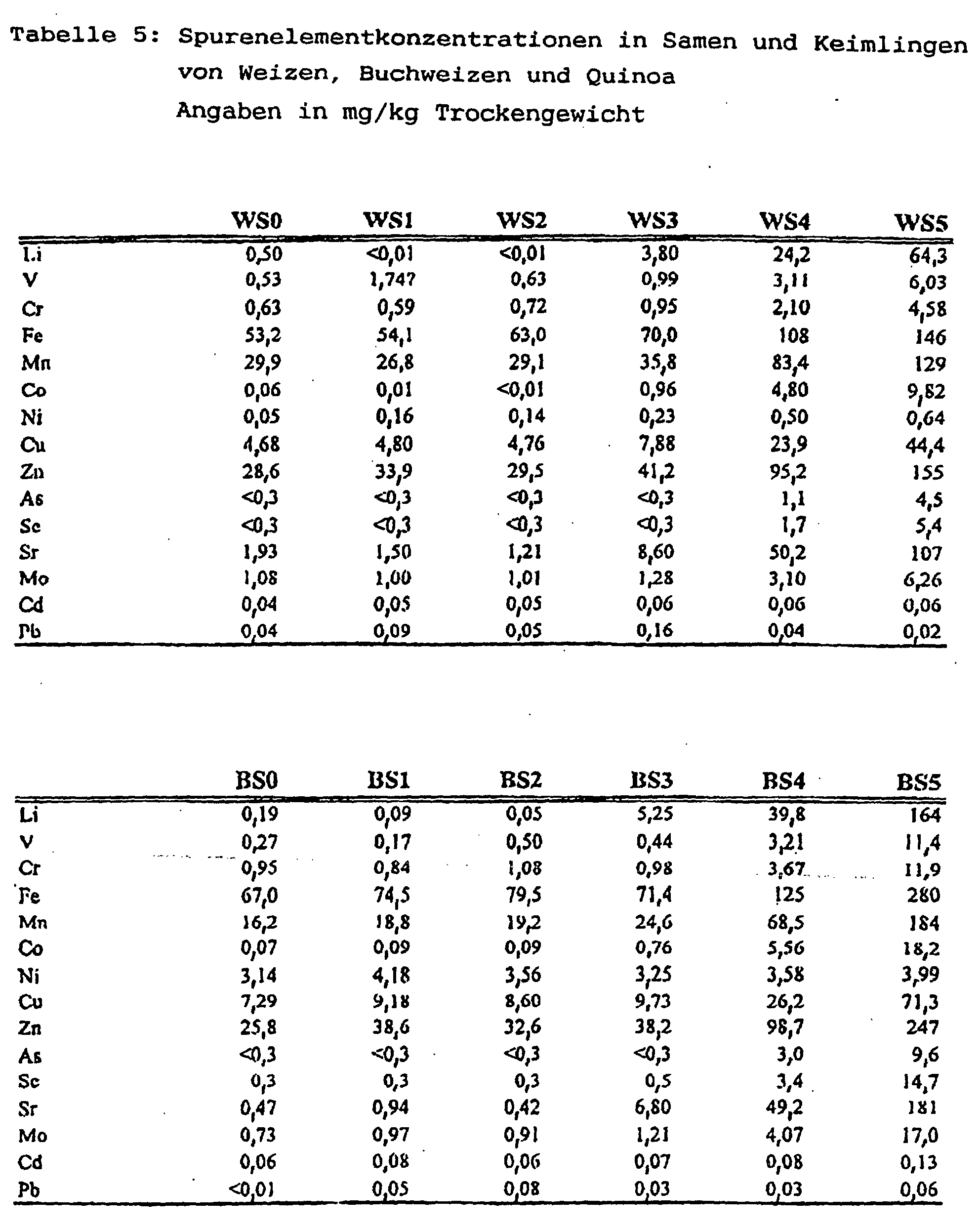

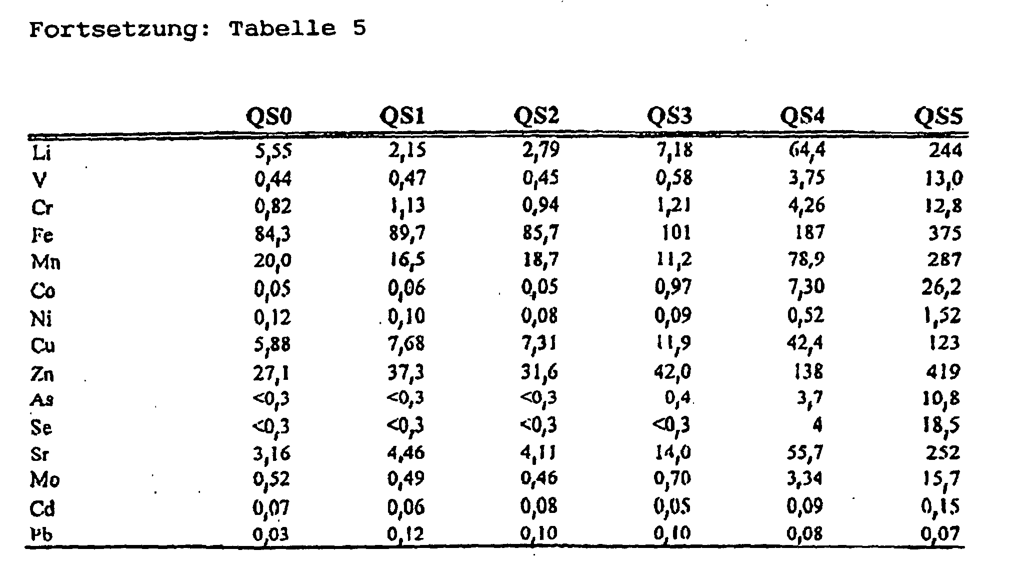

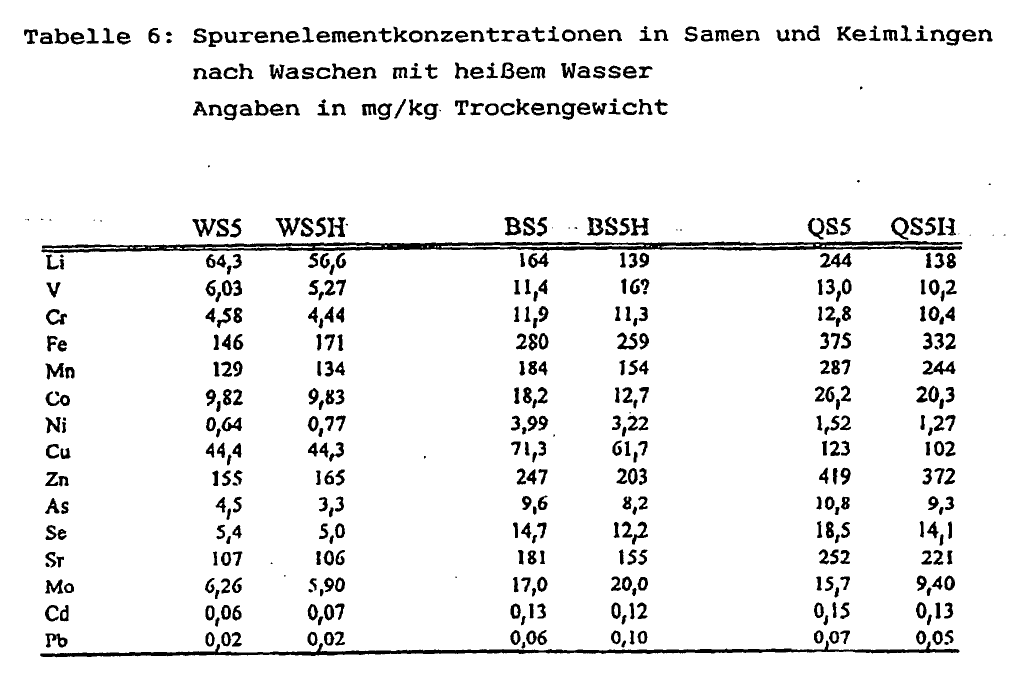

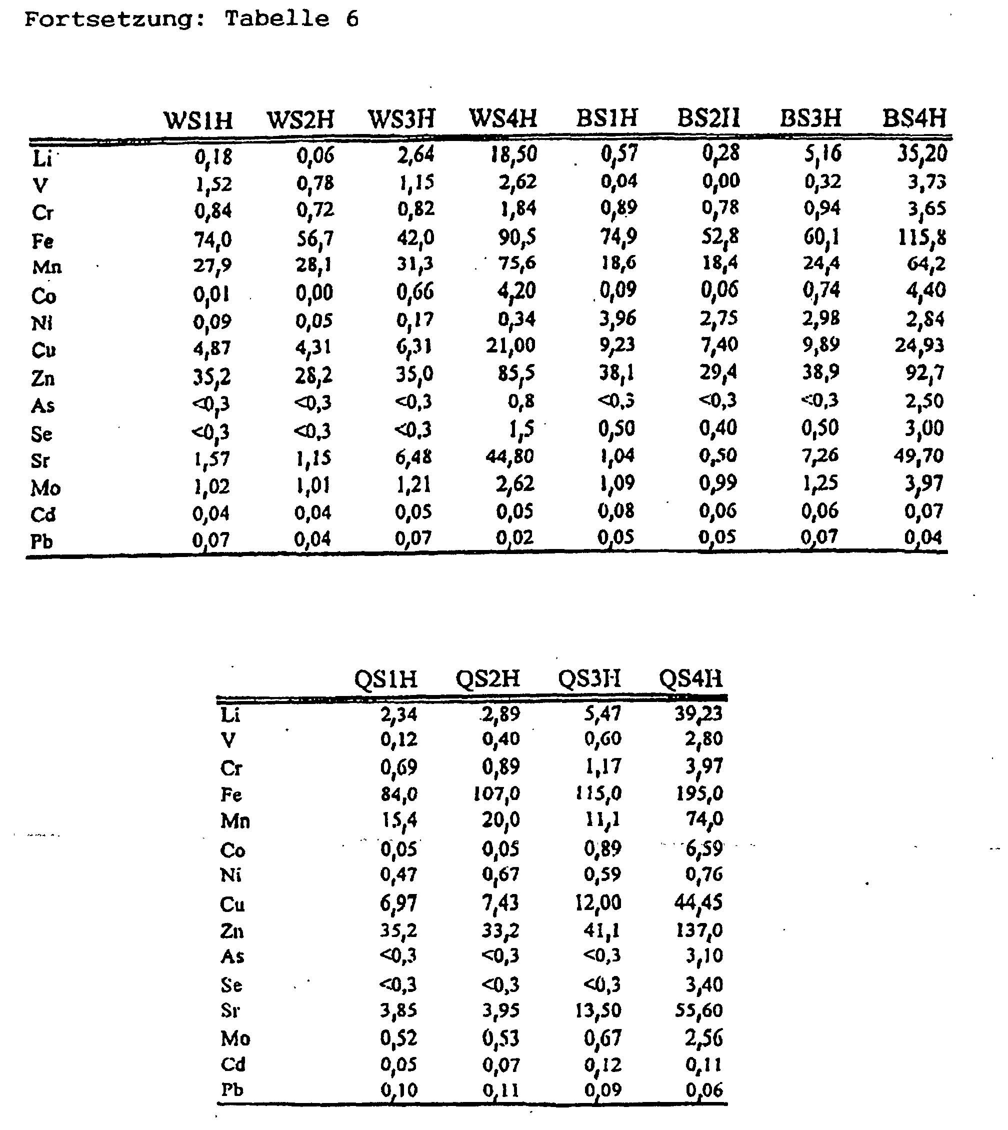

### 2. Bestimmung der Ascorbinsäurekonzentration während der Keimung von Quinoa

Für diese Untersuchung wurde Quinoa-Samen in destilliertem Wasser (QS1, QS1H), Leitungswasser (QS2, QS2H) und Elektrolytlösung 2 (QS4, QS4H) bzw. 3 (QS5, QS5H) zur Keimung gebracht.

### 2.1. Extraktion

Ca. 0,8 g Probe wurden exakt in ein 12 ml Zentrifugenröhrchen eingewogen und mit 5 ml Extraktionslösung (5 % meta-Phosphorsäure, 8 % Essigsäure und 0,005 M EDTA) versetzt. Anschließend wurde das Röhrchen fest verschlossen und 4 min intensiv geschüttelt. Nach der Extraktion wurde die Probenlösung 5 min mit 10000 U/min zentrifugiert. Die klare überständige Lösung wurde vor der HPLC-Analyse durch ein 0,2 µm Cellulosenitratfilter filtriert.

### 2.2. HPLC-Analyse

Die Bestimmung der Vitamin C-Konzentration erfolgte mittels Ionenpaar-reversed-phase-HPLC und UV-Detektion bei 265 nm. Die chromatographischen Parameter sind in Tabelle 7 zusammengefaßt. Quantifiziert wurde mittels externer Eichkurve. Als Standardlösung wurde eine 1000 mg/l Stammlösung (100 mg Vitamin C (Merck p.a.) in 100 ml Extraktionslösung) verwendet. Die Standards für die Eichkurve (20, 50 und 100 mg/l) wurden durch entsprechende Verdünnungen mit der Extraktionslösung hergestellt.

**Tabelle 7:**

| Chromatographische Parameter zur Bestimmung von Vitamin C. | |
|---|---|
| Säule | Hamilton PRP1; 10 µm, 4x250 mm |
| mobile Phase | 0,2 M Na-Acetat; |
| | 5 mM Tetrahexylammoniumbromid (THAB), pH 4,80 |
| Fließgeschwindigkeit | 1,5 ml/min (2000 psi) |
| Injektionsvolumen | 100 µl |
| Detektion | UV 265 nm |

Die Ergebnisse sind in Tabelle 8 dargestellt. Daraus ergibt sich, daß sich die Vitamin C-Konzentration der Keimlinge in Elektrolyt-gekeimten Samen erhöhte.

**Tabelle 8:**

| Vitamin C-Konzentrationen in Samen und Keimlingen von Quinoa | |
|---|---|
| Quinoa | c [mg/100 g] |
| QSO | n.g. |
| QS1 | 6,0 |
| QS1H | 3,5 |
| QS2 | 7,4 |
| QS2H | 2,9 |
| QS4 | 7,9 |
| QS4H | 3,2 |
| QS5 | 6,7 |
| QS5H | 4,2 |
| n.g. = gemessen | |

### 3. Bestimmung der Thiaminkonzentration in Mungobohnen-, Bockshorn- und Rettichkeimlingen

3.1. Samen von Mungobohnen, Bockshornklee und Rettich wurden in Leitungswasser (MS2, BoS2, RS2) und in einer Elektrolytlösung 4 gemäß Tabelle 9 (MS6, BoS6, RS6) 10 bis 12 h eingeweicht. Nach dem Einweichen erfolgte die Keimung bei Raumtemperatur und normalen Tag-Nachtlichtverhältnissen in handelsüblichen Keimgeräten, bestehend aus durchsichtigen übereinander angeordneten Plastikschalen mit Ablaufvorrichtung. Die Keimzeit betrug durchschnittlich 3 Tage; pro Tag wurden die Keimlinge 2-mal gründlich mit Leitungswasser gewaschen. Nach der Keimung wurden die Proben direkt in Plastiksäckchen abgefüllt und eingefroren.

Die Proben der mit Elektrolytlösung 4 behandelten Keimlinge wurden vor der Trocknung 3-mal mit Leitungswasser und anschließend 3-mal mit dreifach destilliertem Wasser gewaschen. Die in Leitungswasser gekeimten Keimlinge wurden direkt ohne zusätzliche Behandlung getrocknet. Die Trocknung erfolgte gemäß Punkt 1.2.

**Tabelle 9:**

| Spurenelement- und Mineralstoffkonzentrationen der für die Keimung zur Thiaminkonzentration verwendeten Elektrolytlösung (Elektrolytlösung 4). (Beim Auflösen in Leitungswasser Bildung eines Niederschlags) | | | |
|---|---|---|---|
| Substanz | c [mg/l] | Element | c [mg/l] |
| Kaliumhydrogenphosphat | 1605,6 | K | 720 |
| Magnesiumphosphat 30% H₂O | 708 | Mg | 100 |
| Zinksulfat x7H₂O | 22 | Zn | 5 |
| Eisen-II-gluconat | 44,7 | Fe | 5 |
| Manganchlorid | 29,5 | Mn | 10 |
| Kupfergluconat | 71,4 | Cu | 10 |
| Natriumselenit | 0,33 | Se | 0,10 |
| Natriummolybdat | 0,25 | Mo | 0,10 |
| Chrom-III-chlorid | 0,51 | Cr | 0,10 |
| Strontiumlactat | 16,85 | Sr | 5 |
| Lithiumcarbonat | 26,75 | Li | 5 |

### 3.2. Analysengang

Von allen Proben wurden jeweils 2 Bestimmungen durchgeführt.

### Hydrolyse und enzymatische Spaltung

Vor der Hydrolyse wurden die getrockneten Proben in einer Analysenmühle (Retsch) homogenisiert. Anschließend wurden 0,50 g Probe exakt in ein 12 ml Zentrifugenröhrchen (Pyrex) eingewogen. Zur Probe wurden 8,5 ml 9,1 M HCl zugegeben; die Röhrchen wurden fest verschlossen und im Wasserbad bei 100°C 30 min unter mehrmaligem Aufschütteln gehalten. Nach Abkühlen der Röhrchen wurden zu der stark sauren Lösung 0,5 ml einer 2,5 M Natriumacetatlösung zugegeben. Der pH-Wert wurde dadurch auf einen Wert von 4,5-4,6 eingestellt. Danach wurden 1 ml der Enzymsuspension (1 g Diastase, Merck 1.03604, in 10 ml H₂O + 1 Tropfen Antischaummittel) zugesetzt und die Probe über Nacht bei Raumtemperatur geschüttelt.

### Oxidation des Thiamins zu Thiochrom

Nach der enzymatischen Spaltung wurde die Probenlösung 20 min mit 3000 U/min zentrifugiert. Von der überständigen Lösung wurde 1 ml in ein Polystyrolröhrchen pipettiert. Zu dieser Lösung wurden 0,5 ml Oxidationsmittel (1 ml einer 1 %igen K₃[Fe(CN)₆]-Lösung + 10 ml 15 % NaOH) zugegeben und gut durchgemischt (5 x Aufziehen mit 0,5 ml Transferpipette). Danach wurde die Oxidationsreaktion durch Neutralisation mit 0,2 ml 40 % Phosphorsäure (H₃PO₄ 80 % : H₂O 1:1 v/v) gestoppt.

### HPLC-Analyse

Vor der chromatographischen Trennung wurden die oxidierten Probelösungen 10 min mit 10000 U/min zentrifugiert. Die klaren Lösungen wurden ohne weitere Behandlung für die Analyse verwendet. Die chromatographischen Parameter sind in Tabelle 10 zusammengefaßt.

**Tabelle 10:**

| Chromatographische Parameter zur Bestimmung von Thiamin | |
|---|---|
| Säule | Hamilton PRP1; 10 µm, 4 x 250 mm |
| mobile Phase | H₂O : MeOH 60:40 v/v |
| Fließgeschwindigkeit | 1 ml/min (2500 psi) |
| Injektionsvolumen | 100 µl |
| Detektor | Fluoreszenz 375/435 |

### Herstellung der Standardlösung:

Als Stammlösung wurden ca. 100 mg Thiaminhydrochlorid (Fluka 95160) in einen 50 ml Maßkolben exakt eingewogen. Dieser wurde bis zur 50 ml-Marke aufgefüllt und gut geschüttelt. Für die Quantifizierung mittels HPLC wurde diese Lösung 1:10000 verdünnt und anschließend analog den Probenlösungen mit Kaliumhexacyanoferrat oxidiert. Für die Erstellung der Eichkurve wurde die oxidierte Lösung noch einmal 1:1 und 1:3 mit Wasser verdünnt.

### 3.3. Ergebnisse

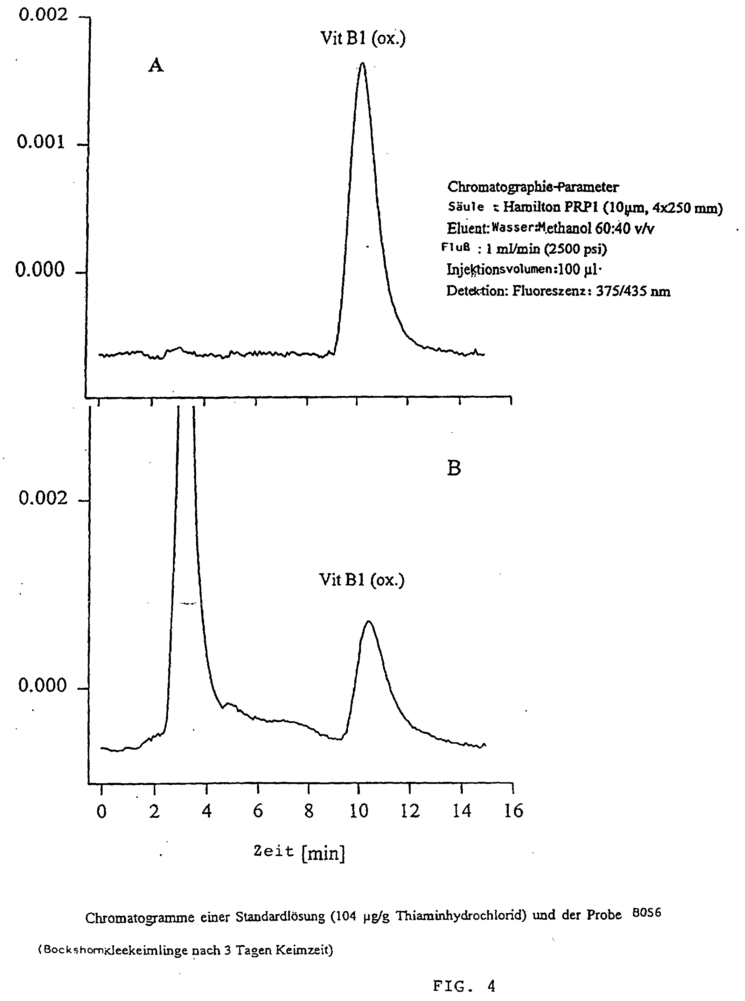

In Fig. 4 sind die Chromatogramme einer Standardlösung (104 µg/l) und der Probe BoS6 (Bockshornkleekeimlinge) zu sehen. Der Peak nach 10 min entspricht dem Thiochrom (oxidierte und stark fluoreszierende Form des Thiamins). Die Thiaminkonzentrationen der einzelnen Proben sind in Tabelle 11 zusammengefaßt.

**Tabelle 11:**

| Thiaminkonzentrationen (bezogen auf Thiaminhydrochlorid) in Keimlingen und Samen; Konzentrationsangaben in mg/100 g. | | | | | |
|---|---|---|---|---|---|
| Mungobohne | | Bockshornklee | | Rettich | |
| Probe | Vit B₁ | Probe | VitB₁ | Probe | Vit B₁ |
| MS0 | 0,03 | BoS0 | <0,02 | RS0 | n.d. |
| MS2 | 0,02 | BoS2 | 0,05 | RS2 | <0,02 |
| MS6 | 0,08 | BoS6 | 0,21 | RS6 | 0,02 |

Ein deutlicher Anstieg der Thiaminkonzentration war während der Keimung von Bockshornklee zu beobachten. Im Samen konnte praktisch kein (<0,02 mg/100 g) Vitamin B₁ nachgewiesen werden, während in den Keimlingen bis zu 0,21 mg/100 g Vitamin B₁ vorhanden waren. Ebenso nahm die Thiaminkonzentration in der Probe Mungobohne während der Keimung zu. Auffallend ist vor allem, daß die Keimlinge der Elektrolytlösung gegenüber den Keimlingen des Leitungswassers eine bedeutend höhere Zunahme aufweisen.

Die Rettichkeimlinge beinhalteten nur eine sehr geringe Menge an Thiamin. Ob die Thiaminkonzentration während der Keimung für RS2 abgenommen hat, konnte aufgrund der fehlenden Analyse des Samens nicht festgestellt werden. Eine Zunahme der Thiaminkonzentration in der Probe RS6 konnte aber deutlich nachgewiesen werden.

## Patentansprüche

1. Verfahren zur Herstellung Elektrolyt-angereicherter Keimlinge. **dadurch gekennzeichnet, daß** keimfähige Samen in eine Elektrolytlösung, enthaltend 1 mg/l oder mehr Zink-, Eisen-, Kaliumund/oder Magnesium-Ionen, 0,5 mg/l oder mehr Kupfer-, Mangan-, Strontium- und/oder Lithium-Ionen und 0,1 mg/l oder mehr Selen-, Molybdän-, Chrom-, Arsen-, Vanadium- und/oder Kobalt-Ionen, eingebracht werden und die Keimlinge in der Elektrolytlösung bei einer geeigneten Temperatur während einer Zeitdauer, die ausreicht, um in den Keimlingen eine Elektrolyt-Anreicherung zu erzielen, inkubiert werden.

2. Verfahren nach Anspruch 1, **dadurch gekennzeichnet, daß** die Keimlinge ausgewählt sind aus Weizen-, Buchweizen-, Quinoa-, Mungobohnen-, Bockshornklee-, Rettich-, Alfalfa-, Mais-, Kürbis-, Roggen-, Gersten-, Reis-, Adzuki-Bohnen-, Erbsen-, Hirse-, Kichererbsen-, Kresse-, Leinsamen-, Linsen-, Senf-, Sesam-, Sojabohnen-, Sonnenblumen- und Amaranthkeimlingen.

3. Verfahren nach Anspruch 1 oder 2, **dadurch gekennzeichnet, daß** eine Elektrolyt-Lösung enthaltend
- 10 mg/l oder mehr, insbesondere 50 mg oder mehr, Zink-, Eisen-, Kalium-, und/oder Magnesium-Ionen,
- 5 mg/l oder mehr, insbesondere 25 mg/l oder mehr, Kupfer-, Mangan-, Strontium- und/oder Lithium-Ionen,
- 1 mg/l oder mehr, insbesondere 5 mg/l oder mehr, Selen-, Molybdän-, Chrom-, Arsen-, Vanadium- und/oder Kobalt-Ionen
zur Anwendung kommt.

4. Verfahren nach einem der Ansprüche 1 bis 3, **dadurch gekennzeichnet, daß** die Inkubation bei einer Temperatur zwischen 10 und 50°C, vorzugsweise zwischen 20°C und 30°C, erfolgt.

5. Verfahren nach einem der Ansprüche 1 bis 4, daß die Inkubation während einer Zeitdauer von etwa 12 bis 240 Stunden, vorzugsweise von etwa 60 bis 100 Stunden, durchgeführt wird.

6. Verfahren nach einem der Ansprüche 1 bis 5, **dadurch gekennzeichnet, daß** die inkubierten Keimlinge gewaschen, getrocknet und gegebenenfalls für den Verkauf geeignet verarbeitet werden.

7. Verfahren nach einem der Ansprüche 1 bis 6, **dadurch gekennzeichnet, daß** eine Elektrolytlösung enthaltend zumindest 0,5 mg/l Kupfer-, 1 mg/l Zink-, 0,1 mg/l Kobalt- und vorzugsweise mindestens 0,1 mg/l Molybdän-, 0,5 mg/l Lithium-, 1 mg/l Selen und 1 mg/l Vanadium-Ionen verwendet wird.

## Claims

1. A method of producing electrolyte-enriched plant embryos, **characterised in that** germinative seeds are introduced into an electrolyte solution containing 1 mg/l or more of zinc, iron, potassium and/or magnesium ions, 0.5 mg/l or more of copper, manganese, strontium and/or lithium ions, and 0.1 mg/l or more of selenium, molybdenum, chromium, arsenic, vanadium and/or cobalt ions, and the plant embryos are incubated in the electrolyte solution at a suitable temperature for a period of time sufficient to attain an electrolyte enrichment within the plant embryos.

2. A method according to claim 1, **characterised in that** the plant embryos are selected from wheat, buckwheat, quinoa, mung bean, fenugreek, radish, alfalfa, maize, squash, rye, barley, rice, adzuki bean, pea, millet, chick pea, cress, linseed, lentil, mustard, sesamum, soybean, sunflower and amaranth embryos.

3. A method according to claim 1 or 2, **characterised in that** an electrolyte solution containing
- 10 mg/l or more, in particular 50 mg/l or more, of zinc, iron, potassium and/or magnesium ions,
- 5 mg/l or more, in particular 25 mg/l or more, of copper, manganese, strontium and/or lithium ions,
- 1 mg/l or more, in particular 5 mg/l or more, of selenium, molybdenum, chromium, arsenic, vanadium and/or cobalt ions are used.

4. A method according to any one of claims 1 to 3, **characterised in that** the incubation is effected at a temperature of between 10 and 50°C, preferably between 20 and 30°C.

5. A method according to any one of claims 1 to 4, **characterised in that** the incubation is carried out for a period of time of approximately 12 to 240 hours, preferably of approximately 60 to 100 hours.

6. A method according to any one of claims 1 to 5, **characterised in that** the incubated plant embryos are washed, dried and optionally processed in a manner suitable for the sale thereof.

7. A method according to any one of claims 1 to 6, **characterised in that** an electrolyte solution containing at least 0.5 mg/l copper, 1 mg/l zinc, 0.1 mg/l cobalt and preferably at least 0.1 mg/l molybdenum, 0.5 mg/l lithium, 1 mg/l selenium and 1 mg/l vanadium ions is used.

## Revendications

1. Procédé de production de plantules enrichies en électrolyte, **caractérisé en ce que** les semences germinatives sont introduites dans une solution d'électrolyte contenant 1 mg/l ou plus d'ions de zinc, de fer, de potassium et/ou de magnésium, 0,5 mg/l ou plus d'ions de cuivre, de manganèse, de strontium et/ou de lithium et 0,1 mg/l ou plus d'ions de sélénium, de molybdène, de chrome, d'arsenic, de vanadium et/ou de cobalt et les plantules dans la solution d'électrolyte sont incubées à une température appropriée pendant une période de temps qui suffit pour produire dans les plantules un enrichissement en électrolyte.

2. Procédé selon la revendication 1, **caractérisé en ce que** les plantules sont choisies parmi des plantules de froment, de sarrasin, de quinoa, d'ambérique, de lotier corniculé, de raifort, de lucerne cultivée, de maïs, de courge, de seigle, d'orge, de riz, de haricots Adzuki, de pois, de millet, de pois chiche, de cresson, de graine de lin, de lentilles, de moutarde, de sésame, de haricots de soja, de tournesol et d'amarante.

3. Procédé selon la revendication 1 ou 2, **caractérisé en ce que** l'on utilise une solution d'électrolyte contenant :
- 10 mg/l ou plus, en particulier 50 mg ou plus, d'ions de zinc, de fer, de potassium et/ou de magnésium,
- 5 mg/l ou plus, en particulier 25 mg/l ou plus, d'ions de cuivre, de manganèse, de strontium et/ou de lithium,
- 1 mg/l ou plus, en particulier 5 mg/l ou plus, d'ions de sélénium, de molybdène, de chrome, d'arsenic, de vanadium et/ou de cobalt.

4. Procédé selon l'une quelconque des revendications 1 à 3, **caractérisé en ce que** l'incubation se fait à une température comprise entre 10°C et 50°C, de préférence entre 20°C et 30°C.

5. Procédé selon l'une quelconque des revendications 1 à 4, **caractérisé en ce que** l'incubation est réalisée au cours d'une période de temps d'environ 12 à 240 heures, de préférence d'environ 60 à 100 heures.

6. Procédé selon l'une quelconque des revendications 1 à 5, **caractérisé en ce que** les plantules incubées sont lavées, séchées et éventuellement traitées de manière appropriée pour la vente.

7. Procédé selon l'une quelconque des revendications 1 à 6, **caractérisé en ce qu'**on utilise une solution d'électrolyte contenant au moins 0,5 mg/l d'ions de cuivre, 1 mg/l d'ions de zinc, 0,1 mg/l d'ions de cobalt et, de préférence au moins 0,1 mg/l d'ions de molybdène, 0,5 mg/l d'ions de lithium, 1 mg/l d'ions de sélénium et 1 mg/l d'ions de vanadium.
